# EUROPÄISCHE PATENTANMELDUNG

(11) **EP 0 936 219 A1**
(43) Veröffentlichungstag der Anmeldung: **18.08.1999**
(21) Anmeldenummer: 99102391.2
(22) Anmeldetag: 08.02.1999
(51) Int. Cl.: C07D 301/12, C07D 303/04, B01J 38/52, B01J 21/20

(54) **Verfahren und Reaktor zur Herstellung eines Epoxids**

(30) Priorität: 11.02.1998 DE 19805552
(71) Anmelder: Linde Aktiengesellschaft, 65189 Wiesbaden (DE)
(72) Erfinder: Ponceau, Marianne Dipl.-Ing., 80634 München (DE); Müller-Markgraf, Wolfgang Dipl.-Chem., 80689 München (DE)

(57) **Zusammenfassung**

Die Erfindung betrifft ein Verfahren und einen Reaktor zur Herstellung eines Epoxids durch chemisches Umsetzen eines Olefins mit Wasserstoffperoxid (H₂O₂) in Gegenwart eines für die Epoxidierung geeigneten Katalysators in einer Flüssigphase eines Reaktors, wobei das H₂O₂ für die Epoxidierung zusammen mit einem ersten Lösungsmittel verwendet wird, der Katalysator im Verlauf der Epoxidierung an katalytischer Aktivität verliert, in Abwesenheit des Olefins mit wäßrigem H₂O₂ regeneriert und nach dem Regenerieren wieder zur Epoxidierung verwendet wird.

Erfindungsgemäß wird beim Regenerieren des Katalysators das H₂O₂ zusammen mit einem zweiten Lösungsmittel verwendet und der Katalysator verbleibt beim Regenerieren im Reaktor. Der Reaktor enthält einen Rührkessel oder eine Rührkesselkaskade oder mindestens einen Festbett- oder Schlaufenreaktor und mindestens eine Zuleitung für Inertgas und eine Ableitung für Sauerstoff und/oder Olefin enthaltendes Gas.

## Beschreibung

Die Erfindung betrifft ein Verfahren und einen Reaktor zur Herstellung eines Epoxids durch chemisches Umsetzen eines Olefins mit Wasserstoffperoxid (H₂O₂) in Gegenwart eines für die Epoxidierung geeigneten Katalysators in einer Flüssigphase eines Reaktors, wobei das H₂O₂ für die Epoxidierung zusammen mit einem ersten Lösungsmittel verwendet wird, der Katalysator im Verlauf der Epoxidierung an katalytischer Aktivität verliert, in Abwesenheit des Olefins mit wäßrigem H₂O₂ regeneriert und nach dem Regenerieren wieder zur Epoxidierung verwendet wird.

Es ist bekannt, Epoxide aus Olefinen herzustellen, indem das Olefin mit Wasserstoffperoxid in Gegenwart eines Titanatome enthaltenden Zeoliths als Katalysator zum Epoxid umgesetzt wird (EP-A 0 100 119). M. G. Clerici, G. Bellussi und U. Romano, J. Catal. 129 (1991) 159 - 157 lehren weiterhin, daß der titanhaltige Zeolith während der Verwendung als Katalysator zur Epoxidierung von Propylenoxid rasch an katalytischer Aktivität verliert. Für die Regenerierung der Aktivität des Katalysators beschreiben Clerici et al. Zwei Möglichkeiten:

Einerseits kann der Zeolith durch Kalzinieren bei 550°C regeneriert werden, andererseits kann die katalytische Aktivität durch Waschen mit Lösungsmitteln bei erhöhter Temperatur, vorzugsweise mit Methanol oder dem zur Epoxidierung verwendeten Lösungsmittel, wiederhergestellt werden. Die Regenerierung durch Kalzinieren hat den Nachteil, daß der Katalysator dazu zunächst getrocknet, dann auf die hohe Kalziniertemperatur erhitzt und anschließend wieder abgekühlt werden muß, was zu einem erheblichen zusätzlichen Energiebedarf und apparativen Aufwand führt. Die Regenerietung durch Waschen mit einem Lösungsmittel ist ein langsamer Prozeß der in der Regel wesentlich mehr Zeit als die eigentliche Epoxidierungsreaktion erfordert.

Eine Verbesserung gegenüber diesen Regeneriermethoden stellt das Regenerieren mit wäßrigem H₂O₂ dar.

In DE 195 28 220 C1 (Degussa) wird beschrieben, wie ein Titan-dotierter Silikalit (z. B. Ti-ZSM5 Zeolith)-Katalysator zur Epoxidation von Olefinen, vorzugsweise von Propylen, mit Wasserstoffperoxid in methanolischer Lösung eingesetzt wird. Es ist bekannt, daß diese Katalysatoren bei der Epoxidierungsreaktion relativ rasch desaktivieren, z. B. nach Degussa-Veröffentlichungen etwa in einem Zeitbereich von ca. 6 - 10 Stunden. In DE 195 28 220 C1 wird deshalb vorgeschlagen, den Katalysator durch Behandlung mit einer wäßrigen Wasserstoffperoxidlösung in Abwesenheit des Olefins zu regenerieren. Dazu ist es notwendig, den desaktivierten Katalysator aus der methanolischen Suspension, wie sie unter Reaktionsbedingungen vorliegt, abzutrennen, in eine rein wäßrige Wasserstoffperoxidlösung zu überführen und den Katalysator in dieser Lösung einige Stunden (typisch sind 4 Stunden für eine vollständige Regeneration) unter Rückfluß zu erhitzen. Anschließend wird der nunmehr regenerierte Katalysator von der Wasserstoffperoxidlösung abgetrennt, mit Wasser/Methanol gewaschen und emeut für die Epoxidationsreaktion eingesetzt. Falls in der Epoxidationsstufe ein erhöhter Wassergehalt toleriert werden kann, ist es optimal möglich, den Katalysator ohne vorherige Abtrennung aus der Wasserstoffperoxidlösung direkt wieder in die Reaktion einzusetzen.

Grundsätzlich beruht diese Art der Regenerierung auf der Beobachtung, daß die aktiven Zentren des Titan-dotierten Zeolithkatalysators durch Oligomere des Produkts blockiert werden, die in geringen Konzentrationen in einer Folgereaktion entstehen und sich mit zunehmender Betriebszeit anreichem. Diese Oligomere werden durch die Behandlung mit Wasserstoffperoxid oxidativ gespalten und die Spaltprodukte gehen in die wäßrige Lösung über.

Die Nachteile des Verfahrens nach DE 195 28 220 C1 können wie folgt zusammengefaßt werden:
- Zur Regenerierung in wäßriger Wasserstoffperoxidlösung muß der Katalysator aus der (methanolischen) Reaktionslösung abgetrennt werden.
- Die vollständige Regeneration erfordert relativ drastische Bedingungen. So muß der Katalysator gewöhnlich ca. 4 Stunden bei der Siedetemperatur der jeweiligen wäßrigen Wasserstoffperoxidlösung unter Rückfluß gekocht werden. Das ist mit hohem Energieaufwand verbunden und die notwendigen Regenerationszeiten sind lang im Vergleich zu den Zeiten die für die eigentliche Synthesereaktion zur Verfügung steht.
- Bei der kontinuierlichen Synthesereaktion kann sich im Gasraum des Reaktors oberhalb der Suspension aus Katalysator, Methanol, Wasser, Wasserstoffperoxid und gelöstem Propylen molekularer Sauerstoff ansammeln. Dieser stammt aus der bekannten Zerfallsreaktion von Wasserstoffperoxid zu Sauerstoff und Wasser. Selbst wenn nur Spuren von Wasserstoffperoxid auf diesem Wege für die Epoxidationsreaktion verlorengehen, kann dies einen aus sicherheitstechnischen Überlegungen unzulässig hohen Gehalt an Sauerstoff in der Propylen-Gasphase im Reaktorkopf ergeben. Es ist deshalb notwendig sicherzustellen, daß die Sauerstoff-Gasphasenkonzentration unter allen Umständen außerhalb des Explosionsbereiches bleibt. Das Problem des Wasserstoffperoxidzerfalls zu Sauerstoff und Wasser und die damit verbundenen Gefahren durch explosionsfähige Gasgemische im Rekatorkopf wurden in den bekannten Veröffentlichungen zu diesem Thema nicht oder nicht ausreichend dargestellt.

Aufgabe der Erfindung ist es daher, diese Nachteile zu vermeiden.

Die Idee zur Erfindung liefert die Überlegung, daß die oxidative Spaltung der desaktivierenden Oligomere durch Wasserstoffperoxid allein nicht ausreicht, um die rasche und vollständige Reaktivierung des Katalysators zu bewirken. Zusätzlich müssen die Spaltprodukte aus den Hohlräumen des Zeoliths transportiert und aufgelöst werden. Dabei spielt die Geschwindigkeit der Poren- und Oberflächendiffusion sowie das Lösungsvermögen des Regenerationsmediums eine entscheidende Rolle.

Die Aufgabe der Erfindung wird gelöst durch ein Verfahren mit den Merkmalen des Anspruchs 1 und durch einen Reaktor mit den Merkmalen des Anspruchs 14. Ausgestaltung der Erfindung sind Gegenstand von Unteransprüchen.

Kennzeichnend an der Erfindung ist, daß beim Regenerieren des Katalysators das H₂O₂ zusammen mit einem zweiten Lösungsmittel verwendet wird und daß der Katalysator beim Regenerieren im Reaktor verbleibt.

Die Verwendung des H₂O₂ zusammen mit dem Lösungsmittel beim Regenerieren ermöglicht eine gleichzeitige oxidative Spaltung der bei der Epoxidierung gebildeten deaktivierenden Oligomere, eine Lösung dieser Spaltprodukte und deren schnelle Entfernung aus den Hohlräumen des Katalysators. Die Regenerierung im Reaktor selbst führt zu einer weiteren Verkürzung der Regenerierzeit und zu einer wesentlichen Vereinfachung des Verfahrens.

Das bei der Epoxidierung verwendete erste Lösungsmittel und/oder das beim Regenerieren verwendete zweite Lösungsmittel kann hauptsächlich Methanol enthalten und es kann vorzugsweise bei der Epoxidierung und beim Regenerieren das gleiche Lösungsmittel verwendet werden. Mit Methanol im Lösungsmittel wird eine besonders schnelle und vollständige Regenerierung erzielt. Mit dem gleichen Lösungsmittel beim Epoxidieren und Regenerieren wird das Verfahren weiter vereinfacht.

Das verwendete H₂O₂ kann aus geeigneten Verbindungen im Reaktor in situ gebildet oder dem Reaktor zugeführt werden. Die Bildung in situ verkürzt die Verweilzeiten des H₂O₂ im Reaktor und mindern so die Bildung von Sauerstoff durch Zerfall des H₂O₂ in Sauerstoff und Wasser. Andererseits ist die Zufuhr des H₂O₂ aus einem Vorratsbehälter verfahrenstechnisch einfacher zu verwirklichen.

Zum Regenerieren in Abwesenheit des Olefins kann das Olefin im Reaktor vor dem Regenerieren weitgehend durch die Epoxidierung abgebaut werden. Der Vorteil besteht darin, daß der Aufwand, das Olefin aus dem Reaktor zu entfernen, geringer wird oder entfallen kann.

Zum Regenerieren in Abwesenheit des Olefins kann das Olefin mit Hilfe eines Inertgasstromes aus der Flüssigphase des Reaktors in eine Gasphase des Reaktors ausgetrieben und aus dem Reaktor gespült werden. Der Vorteil besteht darin, daß das Olefin nahezu vollständig aus den Reaktor entfernt wird und außerdem gleichzeitig der durch den H₂O₂-Zerfall entstandene Sauerstoff mit dem Spülgas den Reaktor verläßt.

Als Inertgas kann Stickstoff verwendet werden. Dies führt zu besonders geringen Betriebskosten im Vergleich zu anderen Inertgasen.

Mit Vorteil kann das Regenerieren bei einer Temperatur zwischen 20 und 80°C, bevorzugt zwischen 30 und 60°C, vorgenommen werden. Auf diese Weise ist es möglich, den Katalysator ohne äußere Wärmezufuhr bei der Temperatur zu regenerieren, die sich beim vorangegangen Epoxidieren einstellt.

Das Regenerieren kann bei einem Druck zwischen 1 und 10 bar, bevorzugt bei einem Druck zwischen 1 und 2 bar, vorgenommen werden. Von Vorteil ist hierbei, daß dieser Druckbereich auch beim Epoxidieren bevorzugt wird, so daß ein Druckwechsel beim Übergang vom Epoxidieren zum Regenerieren und wieder zum Epoxidieren entfallen kann.

Für das Regenerieren kann eine Zeit zwischen 0,5 und 30 Minuten, vorzugsweise zwischen 1 und 10 Minuten, besonders bevorzugt zwischen 1 und 2 Minuten verwendet werden. Die Regeneration erfolgt damit sehr schnell im Vergleich zu einer möglichen Laufzeit zwischen 0,5 und 50 Stunden des Katalysators unter Synthesebedingungen. Dadurch ergeben sich im Vergleich zum Stand der Technik wesentlich höhere mittlere spezifische Umsatzleistungen.

Das Regenerieren oder gegebenenfalls das Spülen gemäß Anspruch 5, kann eingeleitet werden, sobald der Katalysator eine Mindestaktivität für die Epoxdierung unterschreitet oder eine maximale zugelassene Sauerstoffkonzentration in der Gasphase des Reaktors überschritten wird. Der Vorteil besteht darin, daß so eine maximale Laufzeit des Katalysators im Epoxidsynthesemodus und eine hohe Anlagensicherheit erreicht werden.

Alternativ kann neben einer Überwachung der Sauerstoffkonzentration eine Taktlänge für die Epoxidierung zwischen 1 und 20 mittleren Verweilzeiten, vorzugsweise zwischen 1 und 10 Verweilzeiten der Flüssigphase im Reaktor festgelegt werden.

Vorteilhafterweise wird eine mittlere Verweilzeit zwischen 0,5 und 5 Stunden, bevorzugt zwischen 0,5 und 2 Stunden, festgelegt. In diesem Bereich werden je nach Katalysatoreigenschaften die besten Produktausbeuten und -selektivitäten erreicht.

Als für die Epoxidierung geeigneter Katalysator kann ein Titan-Silikalit-Katalysator eingesetzt werden. Von Vorteil ist, daß solche Katalysatoren im Handel erhältlich sein werden.

Kennzeichnend am erfindungsgemäßen Reaktor ist, daß der Reaktor einen Rührkessel oder eine Rührkesselkaskade oder mindestens einen Festbett- oder Schlaufenreaktor enthält und mindestens eine Zuleitung für das Inertgas und eine Ableitung für Sauerstoff und/oder Olefin enthaltendes Gas führt. Der Vorteil des erfindungsgemäßen Reaktors besteht darin, daß mit überraschend einfachen Mitteln ein sicherer Betrieb mit sehr wirkungsvoller Regenerierung des Katalysators im Reaktor ermöglicht wird.

Eine besonders günstige Anwendung findet das erfindungsgemäße Verfahren und der erfindungsgemäße Reaktor bei der Herstellung von Ethylenoxid mit Ethylen als eingesetztem Olefin oder von Propylenoxid mit Propylen als eingesetztem Olefin. Die Verwendung dieser Produkte gewinnt in der chemischen Industrie zunehmend an Bedeutung.

Die Erfindung wird mit zwei Beispielen anhand von zwei Ausführungsformen der Erfindung als Versuchsanlage und mit einer Figur näher erläutert.

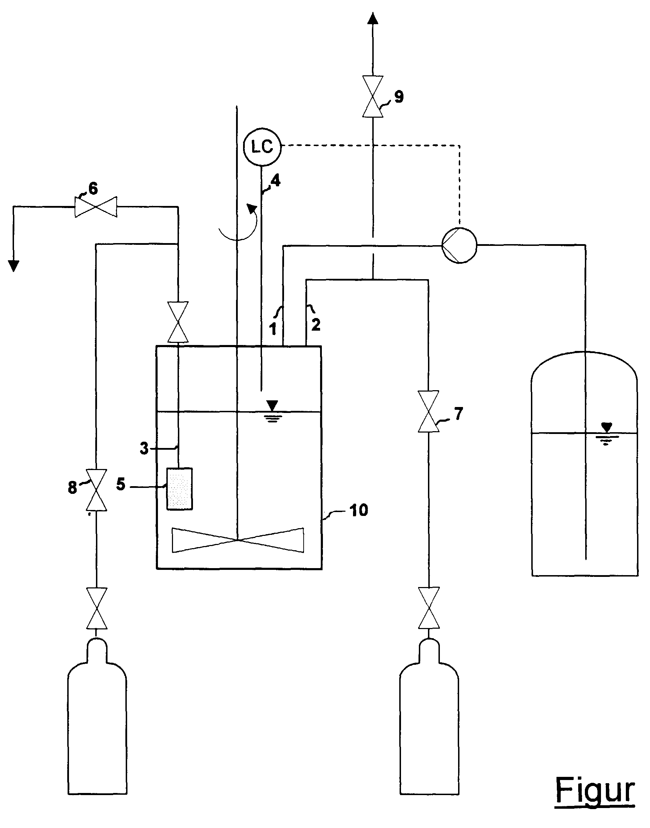

Die Figur zeigt die Erfindung in der Ausführungsform als Versuchsanlage für das Beispiel 2.

### Beispiel 1

### Versuche zur Katalysator-Regenerierung

Die folgenden Ergebnisse aus Batchversuchen zur Propylenoxidsynthese aus Propylen und Wasserstoffperoxid stehen im Zusammenhang mit der Erfindung und führen zu einem kontinuierlichen Verfahren mit instationärer Reaktionsführung:

Die weiter unten folgende Tabelle gibt Reaktionsbedingungen und die Propylenoxid-Ausbeuten bei aufeinanderfolgenden Batchversuchen an. Dazu wird in einem gerührten Druckreaktor eine wäßrige Wasserstoffperoxidlösung und Methanol vorgelegt. Der Titan-Siliciat Katalysator ist in dieser Eduktlösung suspendiert. Die Reaktion wird jeweils gestartet, indem bei 50°C unter Rühren Propylen bis zu einem Druck von 4 bar aufgepreßt wird. Die darauf folgende Druckabnahme durch die Reaktion wird durch Nachdosieren von Propylen ausgeglichen. Nach Ablauf von jeweils 120 Minuten wird der Reaktor geöffnet und die Propylenoxid-Ausbeute gaschromatographisch bestimmt. Der Katalysator wird abfiltriert, und ohne weitere Aufarbeitung in den nächsten Versuch eingesetzt.

Es zeigt sich keine signifikante Abnahme der Produktausbeute, was stark im Gegensatz zu den Angaben in DE 195 28 220 C1 steht. Der Grund hierfür liegt darin, daß der Katalysator zu Beginn eines jeden Batchversuchs durch die methanolische Wasserstoffperoxidlösung vollständig reaktiviert wird, bevor das Propylen in den Reaktor gedrückt wird. Im Unterschied zu DE 195 28 220 C1 wird das Propylen nicht vorgelegt, sondern erst anschließend zugegeben. Daher hat der Katalysator genügend Zeit zur Regeneration durch oxidative Spaltung der desaktivierenden Oligomeren und deren Abtransport und Auflösung im Methanol.

Die unten angegebenen Ausbeuten sind nicht optimiert und haben nur Beispielcharakter. Sie sollen demonstrieren, daß auch bei mehrfach aufeinander folgendem Einsatz des Katalysators eine Deaktivierung des Katalysators nicht erfolgt.

Reaktionsbedingungen in der Reaktionslösung aus H₂O₂ und Methanol:
T = 50°C
p = 4 bar
Verweilzeit = 120 Minuten
Katalysatorkonzentration = 75 g/l Reaktionslösung
H₂O₂-Konzentration = 32,5 g/l Reaktionslösung

### Propylenausbeuten:

| Ansatz Nr. | Ausbeute Propylenoxid % |
|---|---|
| 1 | 80,5 |
| 2 | 83,5 |
| 3 | 83,4 |
| 4 | 78,1 |
| 5 | 78,3 |
| 6 | 80,1 |
| 7 | 82,8 |

### Beispiel 2

Anhand einer Ausführungsform der Erfindung als Versuchsanlage wie in der Figur soll die periodische, instationäre Reaktionsführung, wie sie ähnlich auch in einer Industrieanlage vorgesehen werden kann, beschrieben und dadurch die Erfindung an diesem Beispiel näher erläutert werden.

In einem gerührten Edelstahlreaktor 10 von 300 ml Gesamtvolumen und ca. 150-200 ml Flüssigkeitsvolumen wird bei 50°C und 4 bar Propylenoxid erzeugt. Dazu wird ein Einsatz 1 aus Methanol und wäßrigem Wasserstoffperoxid als Reaktionslösung kontinuierlich in den Reaktor gepumpt. Die Konzentration des Wasserstoffperoxids beträgt zwischen ca. 10 und 200 g H₂O₂/l, in diesem Beispiel 2 etwa 60 g H₂O₂/l Reaktionslösung. Über den Gaseintritt 2 wird ferner Propylen mit einem Druck von 4 bar eingepreßt. Die Produktlösung, die im wesentlichen aus Methanol, Wasser und Propylenoxid besteht, wird über das Tauchrohr 3 entnommen, wobei die Verweilzeit im Reaktor auf einen Wert zwischen ca. 30 min und 5 Stunden, typisch jedoch auf ca. 2 Stunden, eingestellt wird. Dies geschieht durch die Regelung der Entnahmegeschwindigkeit. Eine eingebaute Standregelung 4 steuert die Pumpe für den Einsatz aus Methanol und wäßrigem Wasserstoffperoxid so, daß die über 3 entnommene Menge an Produktlösung gerade durch frischen Einsatz ausgeglichen wird. Um den in der Flüssigphase suspendierten Titan-Silikalit Katalysator zurückzuhalten, ist das Tauchrohr mit einem Filterelement 5 (z. B. Metallfritte) versehen. Wasserstoffperoxid-Umsatz und Propylenoxid-Ausbeute wird am Flüssigkeitsaustritt 3 überwacht. Bei den typischen Ti-dotierten ZSM5-Zeolith-Katalysatoren wird eine Deaktivierung in einem Zeitraum von ca. 6 - 24 Stunden beobachtet, die am Rückgang des H₂O₂-Umsatzes oder der Produktkonzentration sichtbar wird. In diesem Fall wird auf die Regenerierphase umgeschaltet. Dazu wird die Produktentnahme an Ventil 6 gesperrt und die Propylen-Zufuhr an Ventil 7 unterbrochen. Gleichzeitig wird über Ventil 8 Stickstoff durch das Filterelement in die Flüssigphase eingeleitet und mit dem ausgetriebenen Olefin über Ventil 9 wieder abgelassen. Im Versuch zu Beispiel 2 war die Regenerierung des Katalysators bereits bei 1 bis 2 Minuten Verweilzeit in Abwesenheit des Olfefins vollständig erreicht.

Während des Synthesetaktes ist mit zunehmender Deaktivierung des Katalysators eine Anreicherung von Sauerstoff in der Propylen-Gasphase beobachtet worden, die auf Wasserstoffperoxid-Zerfall zu Sauerstoff und Wasser zurückzuführen ist. Obwohl diese Nebenreaktion nur zu geringen Verlusten an Wasserstoffperoxid führt, kann die Anreicherung von Sauerstoff im Extremfall dazu führen, daß die obere Explosionsgrenze unterschritten wird. Mit den periodischen Inertgas-Spültakten werden jedoch die Spuren an Sauerstoff in der Gasphase jedesmal mit ausgetrieben, so daß mit jeder Katalysator-Regeneration auch eine Inertisierung erfolgt.

Nach der Regenerationsphase wird wieder auf Synthese umgeschaltet, indem Ventil 9 und Ventil 8 geschlossen werden und wieder Propylen über Ventil 7 zugeleitet wird. Die Dauer der Synthesephase betrug in Beispiel 2 ca. 4 Verweilzeiten bei ca. 3 Stunden Verweilzeit. Unter diesen Bedingungen konnte keinerlei irreversible Deaktivierung des Katalysators im Langzeitbetrieb festgestellt werden, d.h. die ursprünglich durch die Reaktionsbedingungen eingestellten Produktausbeuten blieben im Langzeitbetrieb konstant. Auch nach versuchsweise sehr langen Synthese-Laufzeiten (mehrere Tage) und vollständiger Katalysator-Deaktivierung konnte mit nur einem einzigen Regenerationstakt die volle Leistungsfähigkeit zurückerhalten werden.

Bei einer Synthesetaktlänge von 4 Verweilzeiten handelt es sich um eine instationäre Reaktionsführung. Somit kann durch Abstimmung der Taktzeiten auf die durch die Katalysatoreigenschaften bestimmte Reaktionskinetik eine Optimierung von Produktausbeuten und -selektivität sowie mittlerer Umsatzleistung erzielt werden.

Falls die Katalysatoreigenschaften sehr lange Laufzeiten im Vergleich zur Verweilzeit erlauben, kann die Dauer der Synthesetakte soweit ausgedehnt werden, daß ein Übergang vom instationären zu einem intermittierend-stationären Betrieb erfolgt.

Das Verfahren kann in der so beschriebenen Betriebsweise auch als Rührkesselkaskade betrieben werden und auch andere Reaktorkonzepte als Rührkessel können eingesetzt werden, solange gewährieistet ist, daß ein zeitweiliges Entfernen des Olefins bei Anwesenheit einer methanolischen Wasserstoffperoxidlösung realisiert werden kann.

Die Umschaltung zwischen Regenerationstakt und Synthesetakt erfolgt zweckmäßig automatisch. Bei gut bekannten Katalysatoreigenschaften können die Taktzeiten fest eingestellt werden, jedoch ist es auch möglich, einen Regenerationstakt in Abhängigkeit von der Katalysatordeaktivierung (Produktanalytik am Reaktorausgang) oder vom Sauerstoffgehalt in der Gasphase (Analytik am Reaktorkopf) auszulösen.

## Patentansprüche

1. Verfahren zur Herstellung eines Epoxids durch chemisches Umsetzen eines Olefins mit Wasserstoffperoxid (H₂O₂) in Gegenwart eines für die Epoxidierung geeigneten Katalysators in einer Flüssigphase eines Reaktors, wobei das H₂O₂ für die Epoxidierung zusammen mit einem ersten Lösungsmittel verwendet wird, der Katalysator im Verlauf der Epoxidierung an katalytischer Aktivität verliert, in Abwesenheit des Olefins mit wäßrigem H₂O₂ regeneriert und nach dem Regenerieren wieder zur Epoxidierung verwendet wird, **dadurch gekennzeichnet**, daß beim Regenerieren des Katalysators das H₂O₂ zusammen mit einem zweiten Lösungsmittel verwendet wird und daß der Katalysator beim Regenerieren im Reaktor verbleibt.

2. Verfahren nach Anspruch 1, dadurch gekennzeichnet, daß das bei Epoxidierung verwendete erste Lösungsmittel und/oder das beim Regenerieren verwendete zweite Lösungsmittel hauptsächlich Methanol enthält und daß vorzugsweise bei der Epoxidierung und beim Regenerieren das gleiche Lösungsmittel verwendet wird.

3. Verfahren nach Anspruch 1 oder 2, dadurch gekennzeichnet, daß das verwendete H₂O₂ aus geeigneten Verbindungen im Reaktor in situ gebildet oder dem Reaktor zugeführt wird.

4. Verfahren nach einem der Ansprüche 1 bis 3, dadurch gekennzeichnet, daß zum Regenerieren in Abwesenheit des Olefins das Olefin im Reaktor vor dem Regenerieren weitgehend durch die Epoxidierung abgebaut wird.

5. Verfahren nach einem der Ansprüche 1 bis 3, dadurch gekennzeichnet, daß zum Regenerieren in Abwesenheit des Olefins das Olefin mit Hilfe eines Inertgasstromes aus der Flüssigphase des Reaktors in eine Gasphase des Reaktors ausgetrieben und aus dem Reaktor gespült wird.

6. Verfahren nach Anspruch 5, dadurch gekennzeichnet, daß als Inertgas Stickstoff verwendet wird.

7. Verfahren nach einem der Ansprüche 1 bis 6, dadurch gekennzeichnet, daß das Regenerieren bei einer Temperatur zwischen 20 und 80°C, bevorzugt zwischen 30 und 60°C, vorgenommen wird.

8. Verfahren nach einem der Ansprüche 1 bis 7, dadurch gekennzeichnet, daß das Regenerieren bei einem Druck zwischen 1 und 10 bar, bevorzugt bei einem Druck zwischen 1 und 2 bar, vorgenommen wird.

9. Verfahren nach einem der Ansprüche 1 bis 8, dadurch gekennzeichnet, daß für das Regenerieren eine Zeit zwischen 0,5 und 30 Minuten, vorzugsweise zwischen 1 und 10 Minuten, besonders bevorzugt zwischen 1 und 2 Minuten, verwendet wird.

10. Verfahren nach einem der Ansprüche 1 bis 9, dadurch gekennzeichnet, daß das Regenerieren, oder gegebenenfalls das Spülen gemäß Anspruch 5, eingeleitet wird, sobald der Katalysator eine Mindestaktivität für die Epoxidierung unterschreitet oder eine maximale zugelassene Sauerstoffkonzentration in der Gasphase des Reaktors überschritten wird.

11. Verfahren nach einem der Ansprüche 1 bis 9, dadurch gekennzeichnet, daß eine Taktlänge für die Epoxidierung zwischen 1 und 20 mittleren Verweilzeiten, vorzugsweise zwischen 1 und 10 Verweilzeiten der Flüssigphase im Reaktor festgelegt wird.

12. Verfahren nach Anspruch 11, dadurch gekennzeichnet, daß eine mittlere Verweilzeit zwischen 0,5 und 5 Stunden, vorzugsweise zwischen 0,5 und 2 Stunden, festgelegt wird.

13. Verfahren nach einem der Ansprüche 1 bis 12, dadurch gekennzeichnet, daß als für die Epoxidierung geeigneter Katalysator ein Titan-Silikalit-Katalysator eingesetzt wird.

14. Reaktor zur Durchführung des Verfahrens nach einem der Ansprüche 1 bis 13, dadurch gekennzeichnet, daß der Reaktor einen Rührkessel oder eine Rührkesselkaskade oder mindestens einen Festbett- oder Schlaufenreaktor enthält und mindestens eine Zuleitung für das Inertgas und eine Ableitung für Sauerstoff und/oder Olefin enthaltendes Gas führt.

15. Anwendung des Verfahrens nach einem der Ansprüche 1 bis 13 und des Reaktors nach Anspruch 14 zur Herstellung von Ethylenoxid mit Ethylen als eingesetztem Olefin oder von Propylenoxid mit Propylen als eingesetztem Olefin.
